# EUROPEAN PATENT APPLICATION

(11) **EP 4 265 288 A1**
(43) Date of publication of application: **25.10.2023**
(21) Application number: 21939246.1
(22) Date of filing: 28.04.2021
(51) Int. Cl.: A61M 15/06, A24F 40/65

(54) **INHALATION DEVICE, CONTROL METHOD, AND PROGRAM**

(71) Applicant: Japan Tobacco Inc., Tokyo 105-6927 (JP)
(72) Inventor: NAGAHAMA, Toru, Tokyo 130-8603 (JP); AOYAMA, Tatsunari, Tokyo 130-8603 (JP); KAWANAGO, Hiroshi, Tokyo 130-8603 (JP); FUJIKI, Takashi, Tokyo 130-8603 (JP); YOSHIDA, Ryo, Tokyo 130-8603 (JP)
(74) Representative: Hoffmann Eitle
(86) International application number: PCT/JP2021/016892
(87) International publication number: WO 2022/230085

(57) **Abstract**

[Problem] To provide a suitable pairing mechanism for an inhalation device. [Solution] This inhalation device is provided with: a generation unit for generating an aerosol; a wireless communication unit for performing wireless communication; and a control unit for controlling wireless communication by the wireless communication unit. The control unit starts transmission of an advertisement when an instruction for performing pairing is issued, and then performs control to continue or restart transmission of the advertisement when the pairing fails or is interrupted and a predetermined condition is met.

## Description

### Technical Field

The present invention relates to an inhaler device, a control method, and a program.

### Background Art

Inhaler devices that generate material to be inhaled by users, such as electronic cigarettes and nebulizers, are widespread. For example, an inhaler device uses a substrate containing an aerosol source for generating an aerosol, a flavor source for imparting a flavor component to the generated aerosol, and the like, to generate the aerosol having the flavor component imparted. The user can taste a flavor by inhaling the aerosol generated by the inhaler device and having the flavor component imparted. Hereinafter, an action of the user inhaling the aerosol is also referred to as a puff or a puff action.

In recent years, it has been studied to provide an inhaler device with a wireless communication function. For example, Patent Literature 1 below discloses that an inhaler device is provided with a Bluetooth (registered trademark) communication function.

### Citation List

### Patent Literature

- Patent Literature 1:: JP 2018-514196 A

### Summary of Invention

### Technical Problem

In some wireless communication standards such as Bluetooth, paired devices communicate with each other. However, in the related art disclosed in Patent Literature 1 and the like, no contrivance is made regarding the pairing of the inhaler device.

The present invention is made in view of the above-described problem, and an object of the present invention is to provide a pairing mechanism suitable for an inhaler device.

### Solution to Problem

To address the above-described problem, an aspect of the present invention provides an inhaler device including a generator that generates an aerosol; a wireless communicator that performs wireless communication; and a controller that controls the wireless communication performed by the wireless communicator. The controller performs control to start transmission of an advertisement when execution of pairing is instructed, and then continue or resume the transmission of the advertisement when the pairing fails or is interrupted and a predetermined condition is satisfied.

The predetermined condition may include that an automatic connection function of the inhaler device is enabled. The automatic connection function may be a function of starting a connection with a paired device when a first operation is performed.

The inhaler device may include a holder that has an opening and holds a substrate inserted through the opening and containing an aerosol source; and a lid that opens and closes the opening. The first operation may be an operation of bringing the lid into a state in which the lid opens the opening.

The execution of the pairing may be instructed by performing a second operation in a state in which the lid closes the opening.

The controller may perform control to interrupt the pairing when a third operation is performed. The third operation may be the operation of bringing the lid into the state in which the lid opens the opening.

The inhaler device may include a memory that stores bonding information on a paired device. The predetermined condition may include that one or more items of the bonding information are stored in the memory.

When a connection request is not received for a predetermined time after the transmission of the advertisement is continued or resumed, the controller may stop the transmission of the advertisement.

When the pairing fails or is interrupted and the predetermined condition is not satisfied, the controller may stop the transmission of the advertisement.

When the pairing is successful, the controller may enable an automatic connection function of the inhaler device. The automatic connection function may be a function of starting a connection with a paired device when a first operation is performed.

The controller may prohibit the pairing in a period other than a period from when the execution of the pairing is instructed to when the pairing ends.

Also, to address the above-described problem, another aspect of the present invention provides a control method for controlling an inhaler device including a generator that generates an aerosol and a wireless communicator that performs wireless communication. The control method includes performing control to start transmission of an advertisement when execution of pairing is instructed, and then continue or resume the transmission of the advertisement when the pairing fails or is interrupted and a predetermined condition is satisfied.

Also, to address the above-described problem, still another aspect of the present invention provides a program for causing a computer that controls an inhaler device including a generator that generates an aerosol and a wireless communicator that performs wireless communication, to execute performing control to start transmission of an advertisement when execution of pairing is instructed, and then continue or resume the transmission of the advertisement when the pairing fails or is interrupted and a predetermined condition is satisfied.

### Advantageous Effects of Invention

As described above, according to the present invention, the pairing mechanism suitable for the inhaler device is provided.

### Brief Description of Drawings

[Fig. 1] Fig. 1 is a schematic diagram of an inhaler device according to an internal configuration example.
[Fig. 2] Fig. 2 is an overall perspective view of the inhaler device according to the present embodiment.
[Fig. 3] Fig. 3 is an overall perspective view of the inhaler device according to the present embodiment in a state in which a stick substrate is held.
[Fig. 4] Fig. 4 is a flowchart presenting an example of a flow of communication control processing executed by the inhaler device according to the present embodiment.
[Fig. 5] Fig. 5 is a flowchart presenting the example of the flow of the communication control processing executed by the inhaler device according to the present embodiment.

### Description of Embodiments

Hereinafter, preferred embodiments of the present invention will be described in detail with reference to the accompanying drawings. In this description and the drawings, structural elements having substantially the same functional configuration are denoted by the same reference numeral, and redundant description thereof will be omitted.

### 1. Configuration example of inhaler device

An inhaler device generates material to be inhaled by a user. In the example described below, the material generated by the inhaler device is an aerosol. Alternatively, the material generated by the inhaler device may be gas.

### (1) Internal configuration example

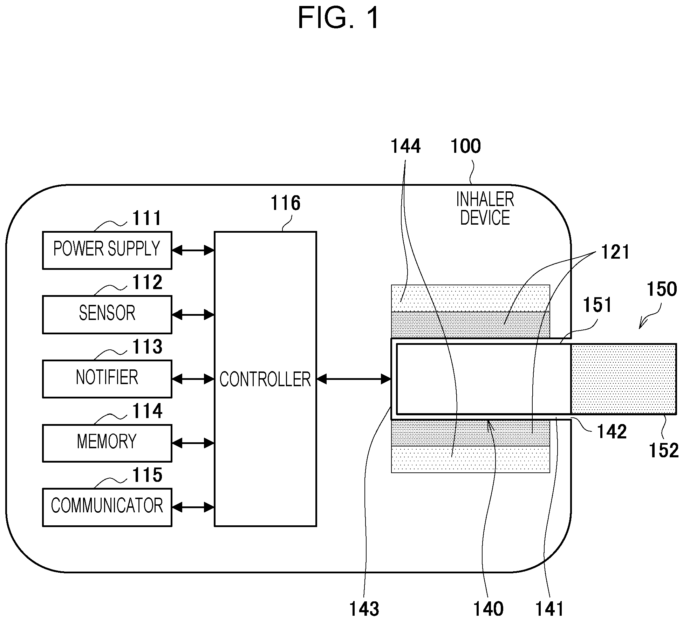

Fig. 1 is a schematic diagram of the inhaler device according to the internal configuration example. As illustrated in Fig. 1, an inhaler device 100 according to the present configuration example includes a power supply 111, a sensor 112, a notifier 113, a memory 114, a communicator 115, a controller 116, a heater 121, a holder 140, and a heat insulator 144.

The power supply 111 stores electric power. The power supply 111 supplies electric power to the structural elements of the inhaler device 100 under the control of the controller 116. The power supply 111 may be a rechargeable battery such as a lithium ion secondary battery.

The sensor 112 acquires various items of information regarding the inhaler device 100. In an example, the sensor 112 may be a pressure sensor such as a microphone condenser, a flow sensor, or a temperature sensor, and acquire a value generated in accordance with the user's inhalation. In another example, the sensor 112 may be an input device that receives information input by the user, such as a button or a switch.

The notifier 113 provides information to the user. The notifier 113 may be a light-emitting device that emits light, a display device that displays an image, a sound output device that outputs sound, or a vibration device that vibrates.

The memory 114 stores various items of information for operation of the inhaler device 100. The memory 114 may be a non-volatile storage medium such as flash memory.

The communicator 115 is a communication interface capable of communication in conformity with any wired or wireless communication standard. Such a communication standard may be, for example, Wi-Fi (registered trademark) or Bluetooth (registered trademark).

The controller 116 functions as an arithmetic processing unit and a control circuit, and controls the overall operations of the inhaler device 100 in accordance with various programs. The controller 116 includes an electronic circuit such as a central processing unit (CPU) and a microprocessor, for example.

The holder 140 has an internal space 141, and holds a stick substrate 150 in a manner partially accommodated in the internal space 141. The holder 140 has an opening 142 that allows the internal space 141 to communicate with outside. The holder 140 holds the stick substrate 150 that is inserted into the internal space 141 through the opening 142. For example, the holder 140 may be a tubular body having the opening 142 and a bottom 143 on its ends, and may define the pillar-shaped internal space 141. The holder 140 also has a function of defining a flow path of air supplied to the stick substrate 150. For example, the bottom 143 has an air inlet hole that is an inlet of air into the flow path. On the other hand, an air outlet hole that is an outlet of the air from the flow path is the opening 142.

The stick substrate 150 includes a substrate 151 and an inhalation port 152. The substrate 151 includes an aerosol source. In this configuration example, the aerosol source is not limited to a liquid, but may be a solid. The stick substrate 150 held by the holder 140 includes the substrate 151 at least partially accommodated in the internal space 141 and the inhalation port 152 at least partially protruding from the opening 142. When the user inhales with the inhalation port 152 protruding from the opening 142 in his/her mouth, air flows into the internal space 141 from the air inlet hole (not illustrated), and the air and an aerosol generated from the substrate 151 reach inside the mouth of the user.

The heater 121 heats the aerosol source to atomize the aerosol source and generate the aerosol. In the example illustrated in Fig. 1, the heater 121 has a film-like shape and surrounds the outer circumference of the holder 140. Subsequently, heat produced from the heater 121 heats the substrate 151 of the stick substrate 150 from the outer circumference, generating the aerosol. The heater 121 produces heat when receiving electric power from the power supply 111. In an example, the electric power may be supplied in response to the sensor 112 detecting a start of the user's inhalation and/or an input of predetermined information. Subsequently, the supply of the electric power may be stopped in response to the sensor 112 detecting an end of the user's inhalation and/or an input of predetermined information.

The heat insulator 144 prevents heat from transferring from the heater 121 to the other structural elements. For example, the heat insulator 144 may be a vacuum heat insulator or an aerogel heat insulator.

The configuration example of the inhaler device 100 has been described above. The inhaler device 100 is not limited to the above configuration, and may be configured in various ways as exemplified below.

In an example, the heater 121 may have a blade-like shape, and may be disposed so that the heater 121 protrudes from the bottom 143 of the holder 140 toward the internal space 141. In this case, the heater 121 having the blade-like shape is inserted into the substrate 151 of the stick substrate 150 and heats the substrate 151 of the stick substrate 150 from its inside. In another example, the heater 121 may be disposed so that the heater 121 covers the bottom 143 of the holder 140. In still another example, the heater 121 may be implemented as a combination of two or more selected from a first heater that covers the outer circumference of the holder 140, a second heater having the blade-like shape, and a third heater that covers the bottom 143 of the holder 140.

In another example, the holder 140 may include an opening/closing mechanism that at least partially opens and closes an outer shell defining the internal space 141. Examples of the opening/closing mechanism include a hinge. In addition, the holder 140 may hold the stick substrate 150 while sandwiching the stick substrate 150 inserted into the internal space 141 by opening and closing the outer shell. In this case, the heater 121 may be at the sandwiching position of the holder 140 and may produce heat while pressing the stick substrate 150.

In addition, means for atomizing the aerosol source is not limited to heating by the heater 121. For example, the means for atomizing the aerosol source may be induction heating.

### (2) Configuration example of appearance

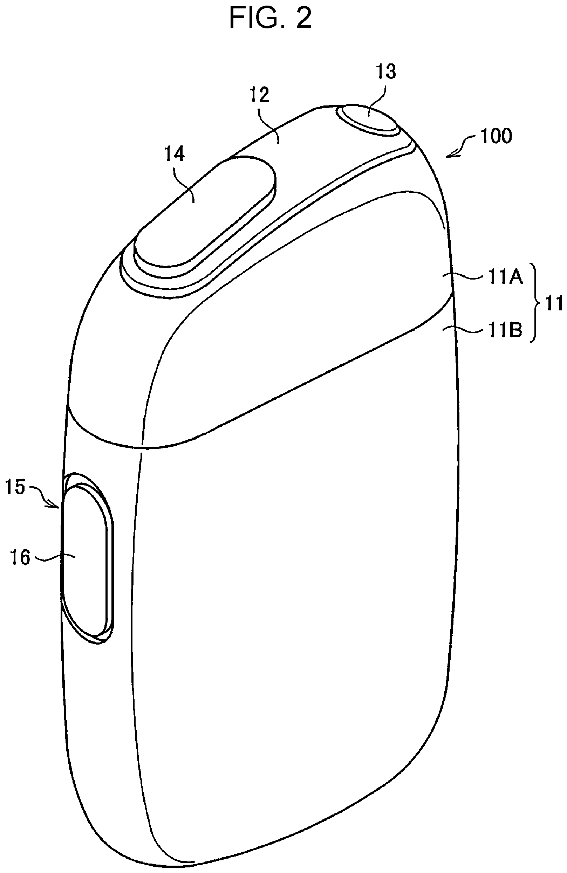

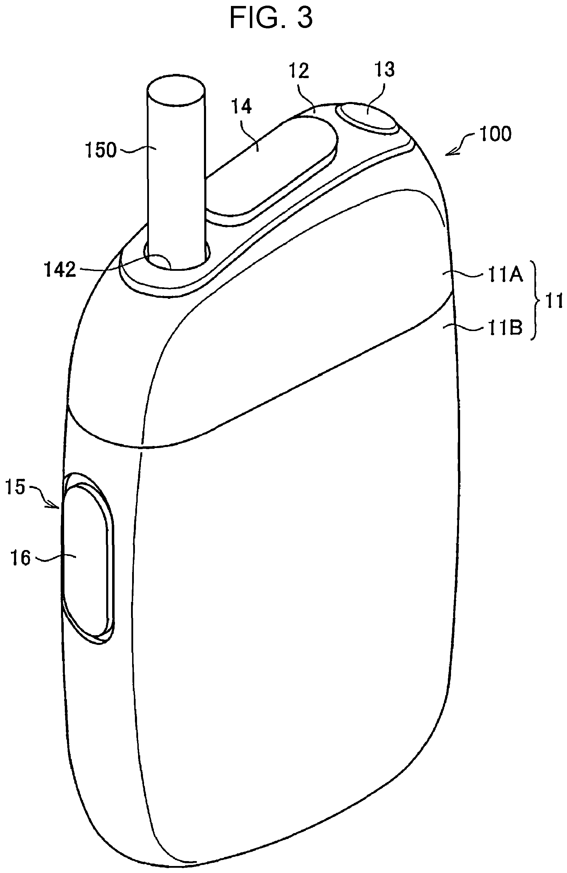

Fig. 2 is an overall perspective view of the inhaler device 100 according to the present embodiment. Fig. 3 is an overall perspective view of the inhaler device according to the present embodiment in a state in which the stick substrate 150 is held.

As illustrated in Fig. 2 and Fig. 3, the inhaler device 100 includes a top housing 11A, a bottom housing 11B, a cover 12, a switch 13, a lid 14, a vent 15, and a cap 16. The top housing 11A and the bottom housing 11B are connected to each other to define an outermost outer housing 11 of the inhaler device 100. The outer housing 11 has a size to fit in a hand of the user. When the user uses the inhaler device 100, the user can hold the inhaler device 100 with his/her hand and inhale a flavor.

The top housing 11A has an opening (not illustrated), and the cover 12 is coupled to the top housing 11A so as to close the opening. As illustrated in Fig. 3, the cover 12 exposes the opening 142 into which the stick substrate 150 can be inserted. The lid 14 is configured to open and close the opening 142. Specifically, the lid 14 is attached to the cover 12 and is configured to be movable along a surface of the cover 12 between a first position at which the lid 14 closes the opening 142 and a second position at which the lid 14 opens the opening 142. Accordingly, the lid 14 can permit or restrict an access of the stick substrate 150 to the inside (the internal space 141 illustrated in Fig. 1) of the inhaler device 100. A state in which the lid 14 is located at the second position and the lid 14 opens the opening 142 is hereinafter also referred to as an open state. A state in which the lid 14 is located at the first position and the lid 14 closes the opening 142 is hereinafter also referred to as a closed state.

The switch 13 is used to switch ON and OFF the operation of the inhaler device 100. For example, when the user operates the switch 13 in a state in which the stick substrate 150 is inserted into the internal space 141 through the opening 142 as illustrated in Fig. 3, electric power is supplied from the power supply 111 to the heater 121, and the stick substrate 150 can be heated without being combusted. When the stick substrate 150 is heated, an aerosol is generated from the aerosol source contained in the stick substrate 150, and a flavor of a flavor source is taken into the aerosol. The user can inhale the aerosol containing the flavor by inhaling a portion (a portion illustrated in Fig. 3, that is, the inhalation port 152) of the stick substrate 150 protruding from the inhaler device 100.

The vent 15 is for introducing air into the internal space 141. The air taken into the inside of the inhaler device 100 from the vent 15 is introduced into the internal space 141 from, for example, the air inlet hole formed in the bottom 143 of the holder 140. The cap 16 is configured to be detachably attached to the bottom housing 11B. When the cap 16 is attached to the bottom housing 11B, the vent 15 is formed between the bottom housing 11B and the cap 16. The cap 16 may have, for example, a through hole, a notch, or the like (not illustrated). In the present description, the longitudinal direction of the inhaler device 100 refers to a direction in which the stick substrate 150 is inserted into the opening 142. Also, in the inhaler device 100 of the present description, a side where a fluid such as air flows in (for example, the vent 15 side) is referred to as an upstream side, and a side where the fluid flows out (for example, the opening 142 side) is referred to as a downstream side.

### 3. Supplement

The heater 121 is an example of a generator that generates an aerosol. The controller 116 controls generation of the aerosol by the heater 121.

The communicator 115 is an example of a wireless communicator that performs wireless communication. There are various wireless communication standards with which the communicator 115 is in conformity. In the present description, it is assumed that the communicator 115 performs wireless communication in conformity with Bluetooth Low Energy (BLE, registered trademark). The controller 116 controls the wireless communication performed by the communicator 115.

### 2. BLE

In BLE, a communication procedure between devices is defined as a protocol. A standardized method of using the protocol is defined as a profile. There are two types of profiles: one standardized for each device characteristic and the other irrespective of the device characteristic. The latter profile is also referred to as a generic profile. The generic profile operates as a lower layer of the profile for each device characteristic. An example of a profile and a protocol defined in BLE will be described below.

### - Generic Access Profile (GAP)

GAP is a generic profile that defines a procedure for enabling communication between devices. In GAP, there are two types of roles of devices: central and peripheral. The central is responsible for control of communication. The peripheral connects to and communicates with the central under the control of the central.

The peripheral first broadcasts an advertising packet (hereinafter, also simply referred to as an advertisement). After transmitting the advertisement, the peripheral waits for reception of a connection request from the central. The peripheral repeats the transmission and reception waiting of the advertisement until the connection request is received. The advertisement may include various items of information such as information specifying whether scanning is performed and information specifying a connection destination.

The central discovers the peripheral by receiving the advertisement. The central determines whether to permit a connection with the peripheral based on the received advertisement. The central may transmit a scan request of requesting transmission of further information to determine whether to permit the connection. The central transmits the connection request to the peripheral permitted for the connection to establish the connection.

### - Generic Attribute Profile (GATT)

GATT is a generic profile that defines data transmission/reception and a data structure. In GATT, there are two types of roles of devices: client and server. The client transmits a request to the server and receives a response from the server. The server receives a request from the client and transmits a response. One of the server and the client may notify the other of information. Typically, the peripheral will be the server and the central will be the client; however, the central may be the server and the peripheral may be the client.

### - Security Manager (SM)

SM is a protocol for establishing security. In GAP, there are two types of roles of devices: initiator and responder. The initiator corresponds to the central in GAP. The responder corresponds to the peripheral in GAP.

In SM, pairing is defined. The pairing is a procedure for generating and exchanging temporary encryption keys. The pairing is performed in the connection established by the procedure defined in GAP.

In SM, bonding is defined. The bonding is a procedure executed after the pairing to generate and exchange permanent encryption keys. The permanent encryption keys are stored in each other's devices. In the subsequent connection, communication is performed using the stored permanent encryption keys, and hence it is not necessary to perform the bonding again.

In SM, encryption re-establishment is defined. The encryption re-establishment is a procedure in which a secure connection is re-established using the permanent encryption keys stored by bonding.

Typically, pairing with bonding is executed. On the other hand, non-bondable pairing may also be executed. In the present description, unless otherwise noted, pairing refers to pairing with bonding and is a concept including both procedures of the above-described pairing and bonding. Also, in the present description, unless otherwise noted, a connection refers to a secure connection using permanent encryption keys generated, exchanged, and stored in bonding.

### 3. Technical features

### (1) Operation mode of inhaler device 100

The inhaler device 100 may operate in various operation modes. Exemplary operation modes for the overall operations of the inhaler device 100 include a sleep mode, a standby mode, and an aerosol generation mode.

The sleep mode is an operation mode in which some of the functions of the inhaler device 100 can be executed. For example, in the sleep mode, among the functions of the sensor 112, only a function of detecting an operation of activating the inhaler device 100 (that is, setting the inhaler device 100 to the standby mode or the aerosol generation mode) may be executable. Accordingly, power consumption can be suppressed to the minimum while the inhaler device 100 can be activated at any timing.

The standby mode is an operation mode in which all functions of the inhaler device 100 can be executed. However, the standby mode is an operation mode in which heating by the heater 121 is not performed. In the standby mode, the inhaler device 100 can execute heating by the heater 121, notification by the notifier 113, and communication by the communicator 115.

The aerosol generation mode is an operation mode in which an aerosol to be inhaled by the user is generated. For example, the aerosol generation mode is an operation mode in which heating by the heater 121 is performed.

The controller 116 switches the operation mode of the inhaler device 100 based on a user's operation. For example, the controller 116 may switch the operation mode of the inhaler device 100 based on an operation on the switch 13 or the lid 14. Of course, the operation mode may be switched based on a user's operation other than the operation on the operation portion. For example, the operation mode may be switched based on detection of a puff. Alternatively, the operation mode may be switched based on that a user's operation is not performed for a predetermined time.

### (2) Automatic connection function

The inhaler device 100 performs various communications with a paired device. An example of a device that is paired with and communicates with the inhaler device 100 is a terminal device operated by the user, such as a smartphone. In an example, the terminal device receives the state of the inhaler device 100, the puff history, and the like, from the inhaler device 100 and notifies the user of the state of the inhaler device 100, the puff history, and the like. In another example, the terminal device transmits information for changing the setting of the inhaler device 100 to the inhaler device 100 and changes the setting of the inhaler device 100. In this way, the inhaler device 100 can provide various services to the user by communicating with the paired device.

One of settings of the inhaler device 100 that can be changed via wireless communication with the terminal device is an automatic connection function. The automatic connection function is a function of starting a connection with a paired terminal device when a first operation is performed.

When the automatic connection function is enabled and when the first operation is performed, the inhaler device 100 starts a connection with the paired terminal device. More specifically, when the automatic connection function is enabled and when the first operation is performed, the communicator 115 starts transmission of an advertisement. Accordingly, the inhaler device 100 can establish a connection with the paired terminal device that has received the advertisement and communicate with the paired terminal device.

In contrast, when the automatic connection function is disabled, the inhaler device 100 does not start a connection with the paired terminal device even though the first operation is performed. More specifically, when the automatic connection function is disabled, the communicator 115 does not start transmission of an advertisement even though the first operation is performed. With such a configuration, power consumption of the inhaler device 100 can be suppressed.

An example of the first operation is an operation of bringing the lid 14 into the state in which the lid 14 opens the opening 142. In the open state, it is expected that the stick substrate 150 is inserted and heating is started immediately thereafter. It is considered that communication is often performed between the inhaler device 100 and the terminal device before and after heating. In this regard, with such a configuration, since a connection can be established before a timing at which communication is performed, usability can be improved.

### (3) BLE communication by inhaler device 100

The inhaler device 100 can perform pairing with an unpaired terminal device, establish a connection, and perform communication. When the pairing is successful, the memory 114 stores bonding information on the paired terminal device. The bonding information includes a permanent encryption key generated in bonding. Accordingly, in the subsequent connection, the inhaler device 100 can perform secure communication with the paired terminal device using the stored bonding information.

When execution of pairing is instructed, the controller 116 controls the communicator 115 to start transmission of an advertisement. When the controller 116 receives a connection request from an unpaired terminal device that has received the advertisement, the controller 116 performs pairing with the terminal device, establishes a connection, and performs communication. During the attempt of the pairing, the operation mode of the inhaler device 100 may be the standby mode.

### - When pairing is not successful

When the pairing fails or is interrupted and a predetermined condition is satisfied, the controller 116 controls the communicator 115 to continue or resume the transmission of the advertisement. The interruption of the pairing indicates that the pairing processing is stopped based on a user's operation. The failure of the pairing indicates that the pairing fails due to a cause other than the user's operation. An example of such a cause is that the connection request is not received for a predetermined time. The transmission of the advertisement is resumed when the transmission of the advertisement is stopped due to the reception of the connection request. The transmission of the advertisement is continued when the transmission of the advertisement is continued without receiving the connection request. With such a configuration, even though pairing is not successful, as long as there is a paired terminal device in a range in which an advertisement is receivable, it is possible to establish a connection and perform communication under the predetermined condition.

However, when the connection request is not received for a predetermined time after the transmission of the advertisement is continued or resumed, the controller 116 stops the transmission of the advertisement. When a paired terminal device is not present in the range in which an advertisement is receivable, the connection request is not returned from the paired terminal device even though the inhaler device 100 transmits the advertisement. In this regard, with such a configuration, since useless transmission of an advertisement is stopped, power consumption can be suppressed. The controller 116 may stop the transmission of the advertisement and switch the operation mode to the sleep mode. With such a configuration, power consumption can be further suppressed.

The predetermined condition is that there is a possibility of communication with a paired terminal device. With such a configuration, since an advertisement is transmitted only when there is a possibility of communication with the paired terminal device, power consumption of the inhaler device 100 can be suppressed.

In an example, the predetermined condition may include that the automatic connection function of the inhaler device 100 is enabled. When the automatic connection function is enabled, it is considered that the user desires to establish an automatic connection with the paired terminal device. In this regard, with such a configuration, since a connection is automatically attempted without bringing the state of the lid 14 into the open state, usability can be improved.

In another example, the predetermined condition may include one or more pieces of bonding information stored in the memory 114. In other words, the predetermined condition may include already being paired with one or more terminal devices.

In contrast, when the pairing fails or is interrupted and the predetermined condition is not satisfied, the controller 116 stops the transmission of the advertisement. That is, the controller 116 does not transmit the advertisement when the pairing fails or is interrupted and there is no possibility of communication with the paired terminal device. With such a configuration, power consumption can be suppressed. The controller 116 may stop the transmission of the advertisement and switch the operation mode to the sleep mode. With such a configuration, power consumption can be further suppressed.

### - When pairing is successful

When the pairing is successful, the controller 116 may enable the automatic connection function. In order to receive provision of a service by the paired terminal device, it is desirable to automatically establish a connection between the inhaler device 100 and the paired terminal device and start communication. In this regard, with such a configuration, it is possible to reduce the time and effort for the user to manually enable the automatic connection function.

### - Operation of instructing execution/interruption of pairing

The execution of the pairing may be instructed by performing a second operation in the state in which the lid 14 closes the opening 142. That is, when the second operation is performed in the state in which the lid 14 closes the opening 142, the controller 116 may perform control to start the transmission of the advertisement. An example of the second operation is pressing the switch 13 in a predetermined pressing pattern. The pressing pattern is defined by at least one of the number of times the switch 13 is pressed, the interval at which the switch 13 is pressed (that is, the length of time from the end of pressing of the switch 13 to the next pressing of the switch 13), and the time during which the switch 13 is pressed (that is, the length of time during which the switch 13 is pressed). With such a configuration, an advertisement for pairing is transmitted at a timing at which the state of the lid 14 is the closed state, that is, at a timing at which the advertisement is not transmitted even though the automatic connection function is enabled. In this way, it is possible to prevent a situation in which pairing with an unpaired terminal device and establishment of a connection with a paired terminal device conflict with each other.

The interruption of the pairing may be instructed by performing a third operation. That is, the controller 116 may perform control to interrupt the pairing when the third operation is performed. In the open state, it is expected that the stick substrate 150 is inserted and heating is started immediately thereafter. It is considered that communication is often performed between the inhaler device 100 and the terminal device before and after heating. In this regard, with such a configuration, pairing with an unpaired terminal device is interrupted, and priority is given to communication with a paired terminal device. Thus, since it is possible to shorten the time until communication with the terminal device is available, usability can be improved.

Preferably, the third operation is the same as the first operation. This is because, as in the case where the first operation is performed, after the third operation is performed, an advertisement for establishing a connection with the paired terminal device is transmitted. That is, an example of the third operation is the operation of bringing the lid 14 into the state in which the lid 14 opens the opening 142. Of course, the third operation is not limited to such an example, and may be, for example, pressing the switch 13 in a predetermined pressing pattern. In this case, the user can interrupt the pairing even when he/she is not about to insert the stick substrate 150 and start heating.

### - Permission/prohibition of pairing

The controller 116 permits the pairing in a first period from when the execution of the pairing is instructed to when the pairing ends. Here, the end of the pairing includes both a case where the pairing ends successfully and a case where the pairing ends due to interruption or failure. When pairing is permitted, based on a connection request from an unpaired terminal device, the controller 116 executes pairing with the terminal device. With such a configuration, pairing can be performed in the first period.

In the first period, the controller 116 controls the communicator 115 to transmit an advertisement. Thus, a connection request may be received not only from an unpaired terminal device but also from a paired terminal device. In this case, the controller 116 may give priority to pairing with the unpaired terminal device over establishment of a connection and communication with the paired terminal device. With such a configuration, even though there is a paired terminal device in the range in which an advertisement is receivable, it is possible to preferentially execute pairing with an unpaired terminal device.

In contrast, the controller 116 prohibits pairing in a second period that is a period other than the first period from when the execution of the pairing is instructed to when the pairing ends. When pairing is prohibited, even though a connection request is received from an unpaired terminal device, the controller 116 does not execute pairing with the terminal device. Then, when a connection request is received from a paired terminal device, the controller 116 establishes a connection with the terminal device and communicates with the terminal device. With such a configuration, since an unspecified terminal device is prevented from being connected to the inhaler device 100 in the second period, security can be improved.

Here, an advertisement may be transmitted both in the first period and in the second period. The advertisement transmitted in the first period and the advertisement transmitted in the second period may be the same. Of course, the advertisement transmitted in the first period and the advertisement transmitted in the second period may be different. In an example, the advertisement transmitted in the first period may include information for permitting a connection with an unpaired terminal device, and the advertisement transmitted in the second period may include information for permitting a connection with a paired terminal device.

### (4) Flow of processing

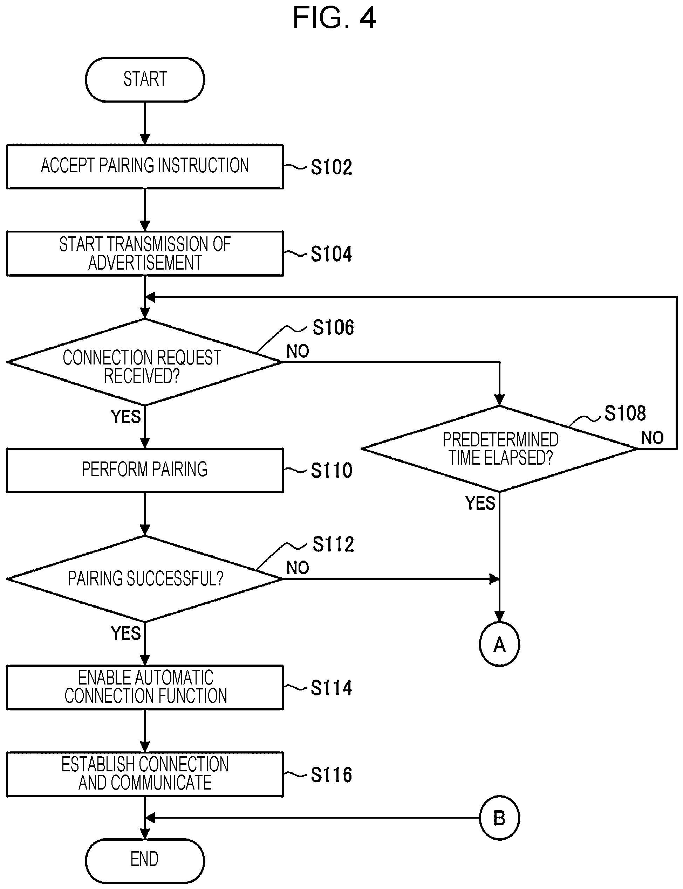

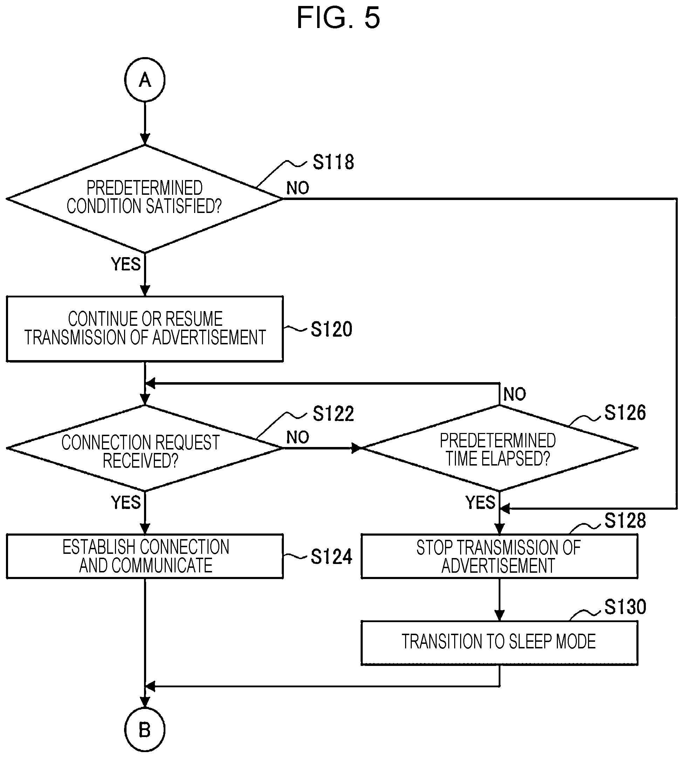

Fig. 4 and Fig. 5 are flowcharts presenting an example of a flow of communication control processing executed by the inhaler device 100 according to the present embodiment.

As illustrated in Fig. 4, first, the inhaler device 100 accepts a user's operation of instructing execution of pairing (step S 102). An example of the user's operation of instructing execution of pairing is pressing the switch 13 in a predetermined pressing pattern in the state in which the lid 14 closes the opening 142.

Next, the inhaler device 100 starts transmission of an advertisement (step S104). Then, the inhaler device 100 periodically transmits the advertisement and waits for reception of a connection request every time the inhaler device 100 transmits the advertisement. The inhaler device 100 transmits the advertisement in a state in which pairing is permitted.

Next, the inhaler device 100 determines whether a connection request has been received (step S106).

When it is determined that the connection request has not been received (step S106: NO), the inhaler device 100 determines whether a predetermined time has elapsed since the transmission of the advertisement is started (step S108).

When it is determined that the predetermined time has not elapsed since the transmission of the advertisement is started (step S108: NO), the processing returns to step S106 again. In contrast, when it is determined that the predetermined time has elapsed since the transmission of the advertisement is started (step S108: YES), the processing proceeds to step S118.

When it is determined that the connection request has been received (step S106: YES), the inhaler device 100 performs pairing with the terminal device that is the transmission source of the connection request (step S110).

Then, the inhaler device 100 determines whether the pairing is successful (step S112).

When the pairing is successful (step S112: YES), the inhaler device 100 enables the automatic connection function (step S114).

Then, the inhaler device 100 establishes a connection with the paired terminal device and communicates with the paired terminal device (step S116). When the communication ends, the inhaler device 100 disconnects the connection. Then, the processing ends.

When the pairing fails or is interrupted (step S112: NO), as illustrated in Fig. 5, the inhaler device 100 determines whether a predetermined condition is satisfied (step S118). The predetermined condition includes, for example, that the inhaler device 100 stores one or more items of bonding information and that the automatic connection function is enabled.

When it is determined that the predetermined condition is not satisfied (step S118: NO), the processing proceeds to step S128.

In contrast, when it is determined that the predetermined condition is satisfied (step S118: YES), the inhaler device 100 continues or resumes the transmission of the advertisement (step S120). Then, the inhaler device 100 periodically transmits the advertisement and waits for reception of a connection request every time the inhaler device 100 transmits the advertisement. The inhaler device 100 transmits the advertisement in a state in which pairing is prohibited.

Next, the inhaler device 100 determines whether a connection request has been received (step S122).

When it is determined that the connection request has been received (step S122: YES), the inhaler device 100 establishes a connection with the terminal device that is the transmission source of the connection request and communicates with the terminal device (step S124). Here, the inhaler device 100 establishes a connection with the paired terminal device and communicates with the paired terminal device. When the communication ends, the inhaler device 100 disconnects the connection. Then, the processing ends.

When it is determined that the connection request has not been received (step S124: NO), the inhaler device 100 determines whether a predetermined time has elapsed since the transmission of the advertisement is continued or resumed (step S126).

When it is determined that the predetermined time has not elapsed since the transmission of the advertisement is continued or resumed (step S126: NO), the processing returns to step S122 again.

In contrast, when it is determined that the predetermined time has elapsed since the transmission of the advertisement is continued or resumed (step S126: YES), the inhaler device 100 stops the transmission of the advertisement (step S128).

Then, the inhaler device 100 transitions to the sleep mode (step S130). Then, the processing ends.

### 4. Supplement

Although the preferred embodiments of the present invention have been described in detail with reference to the accompanying drawings, the present invention is not limited to these examples. It will be apparent to those who have ordinary knowledge in the technical field to which the present invention pertains that various changes and modifications can be made within the scope of the technical idea as defined in the appended claims. It is to be understood that the changes and modifications also obviously pertain to the technical scope of the present invention.

For example, although the example in which the inhaler device 100 heats the aerosol source by heating the stick substrate 150 formed as a solid to generate the aerosol has been described in the above-described embodiment, the present invention is not limited to this example. For example, the inhaler device 100 may generate an aerosol by heating an aerosol source as a liquid. Also, the inhaler device 100 may generate an aerosol by applying vibration to an aerosol source as a liquid.

For example, although the example in which the inhaler device 100 performs wireless communication in conformity with BLE has been described in the above-described embodiment, the present invention is not limited to this example. The wireless communication standard with which the inhaler device 100 is in conformity may be any wireless communication standard with which communication is performed after pairing is performed. Examples of such wireless communication standards other than BLE include Bluetooth and a wireless local area network (LAN).

Note that the series of processing performed by each device described in the present description may be implemented using any of software, hardware, and a combination of software and hardware. A program constituting the software is stored in advance in, for example, a recording medium (non-transitory media) provided inside or outside each device. For example, each program is read into a RAM and executed by a processor such as a CPU at the time of execution by a computer that controls each device described in the present description. The recording medium is, for example, a magnetic disk, an optical disk, a magnetooptical disk, a flash memory, or the like. The computer program may be distributed via a network, for example, without using a recording medium.

Also, the processing described using the flowcharts and the sequence diagrams in the present description do not have to be executed in the illustrated order. Some of the processing steps may be performed in parallel. Also, an additional processing step may be employed, or the processing steps may be partially omitted.

The following configurations also pertain to the technical scope of the present invention.
(1) An inhaler device comprising:
   a generator that generates an aerosol;
   a wireless communicator that performs wireless communication; and
   a controller that controls the wireless communication performed by the wireless communicator,
   wherein the controller performs control to start transmission of an advertisement when execution of pairing is instructed, and then continue or resume the transmission of the advertisement when the pairing fails or is interrupted and a predetermined condition is satisfied.
(2) The inhaler device according to said (1),
   wherein the predetermined condition includes that an automatic connection function of the inhaler device is enabled, and
   wherein the automatic connection function is a function of starting a connection with a paired device when a first operation is performed.
(3) The inhaler device according to said (2), comprising:
   a holder that has an opening and holds a substrate inserted through the opening and containing an aerosol source; and
   a lid that opens and closes the opening,
   wherein the first operation is an operation of bringing the lid into a state in which the lid opens the opening.
(4) The inhaler device according to said (3),
   wherein the execution of the pairing is instructed by performing a second operation in a state in which the lid closes the opening.
(5) The inhaler device according to said (4),
   wherein the controller performs control to interrupt the pairing when a third operation is performed, and
   wherein the third operation is the operation of bringing the lid into the state in which the lid opens the opening.
(6) The inhaler device according to any one of said (1) to (5), comprising:
   a memory that stores bonding information on a paired device,
   wherein the predetermined condition includes that one or more items of the bonding information are stored in the memory.
(7) The inhaler device according to any one of said (1) to (6),
   wherein, when a connection request is not received for a predetermined time after the transmission of the advertisement is continued or resumed, the controller stops the transmission of the advertisement.
(8) The inhaler device according to any one of said (1) to (7),
   wherein, when the pairing fails or is interrupted and the predetermined condition is not satisfied, the controller stops the transmission of the advertisement.
(9) The inhaler device according to any one of said (1) to (8),
   wherein, when the pairing is successful, the controller enables an automatic connection function of the inhaler device, and
   wherein the automatic connection function is a function of starting a connection with a paired device when a first operation is performed.
(10) The inhaler device according to any one of said (1) to (9),
   wherein the controller prohibits the pairing in a period other than a period from when the execution of the pairing is instructed to when the pairing ends.
(11) A control method for controlling an inhaler device including a generator that generates an aerosol and a wireless communicator that performs wireless communication, the control method comprising:
   performing control to start transmission of an advertisement when execution of pairing is instructed, and then continue or resume the transmission of the advertisement when the pairing fails or is interrupted and a predetermined condition is satisfied.
(12) A program for causing a computer that controls an inhaler device including a generator that generates an aerosol and a wireless communicator that performs wireless communication, to execute:
   performing control to start transmission of an advertisement when execution of pairing is instructed, and then continue or resume the transmission of the advertisement when the pairing fails or is interrupted and a predetermined condition is satisfied.

### Reference Signs List

- 100: inhaler device
- 111: power supply
- 112: sensor
- 113: notifier
- 114: memory
- 115: communicator
- 116: controller
- 121: heater
- 140: holder
- 141: internal space
- 142: opening
- 143: bottom
- 144: heat insulator
- 150: stick substrate
- 151: substrate
- 152: inhalation port
- 11: outer housing
- 11A: top housing
- 11B: bottom housing
- 12: cover
- 13: switch
- 14: lid
- 15: vent
- 16: cap

## Claims

1. An inhaler device comprising:
a generator that generates an aerosol;
a wireless communicator that performs wireless communication; and
a controller that controls the wireless communication performed by the wireless communicator,
wherein the controller performs control to start transmission of an advertisement when execution of pairing is instructed, and then continue or resume the transmission of the advertisement when the pairing fails or is interrupted and a predetermined condition is satisfied.

2. The inhaler device according to claim 1,
wherein the predetermined condition includes that an automatic connection function of the inhaler device is enabled, and
wherein the automatic connection function is a function of starting a connection with a paired device when a first operation is performed.

3. The inhaler device according to claim 2, comprising:
a holder that has an opening and holds a substrate inserted through the opening and containing an aerosol source; and
a lid that opens and closes the opening,
wherein the first operation is an operation of bringing the lid into a state in which the lid opens the opening.

4. The inhaler device according to claim 3,
wherein the execution of the pairing is instructed by performing a second operation in a state in which the lid closes the opening.

5. The inhaler device according to claim 4,
wherein the controller performs control to interrupt the pairing when a third operation is performed, and
wherein the third operation is the operation of bringing the lid into the state in which the lid opens the opening.

6. The inhaler device according to any one of claims 1 to 5, comprising:
a memory that stores bonding information on a paired device,
wherein the predetermined condition includes that one or more items of the bonding information are stored in the memory.

7. The inhaler device according to any one of claims 1 to 6,
wherein, when a connection request is not received for a predetermined time after the transmission of the advertisement is continued or resumed, the controller stops the transmission of the advertisement.

8. The inhaler device according to any one of claims 1 to 7,
wherein, when the pairing fails or is interrupted and the predetermined condition is not satisfied, the controller stops the transmission of the advertisement.

9. The inhaler device according to any one of claims 1 to 8,
wherein, when the pairing is successful, the controller enables an automatic connection function of the inhaler device, and
wherein the automatic connection function is a function of starting a connection with a paired device when a first operation is performed.

10. The inhaler device according to any one of claims 1 to 9,
wherein the controller prohibits the pairing in a period other than a period from when the execution of the pairing is instructed to when the pairing ends.

11. A control method for controlling an inhaler device including a generator that generates an aerosol and a wireless communicator that performs wireless communication, the control method comprising:
performing control to start transmission of an advertisement when execution of pairing is instructed, and then continue or resume the transmission of the advertisement when the pairing fails or is interrupted and a predetermined condition is satisfied.

12. A program for causing a computer that controls an inhaler device including a generator that generates an aerosol and a wireless communicator that performs wireless communication, to execute:
performing control to start transmission of an advertisement when execution of pairing is instructed, and then continue or resume the transmission of the advertisement when the pairing fails or is interrupted and a predetermined condition is satisfied.
